(19) Europäisches Patentamt
European Patent Office
Office européen des brevets

(11) Veröffentlichungsnummer: **0 007 516**
**B1**

(12) # EUROPÄISCHE PATENTSCHRIFT

(45) Veröffentlichungstag der Patentschrift: **28.10.81**

(51) Int. Cl.³: **C 07 J 1/00,** C 07 C 49/623

(21) Anmeldenummer: **79102371.6**

(22) Anmeldetag: **11.07.79**

(54) **7-Alpha-Methyl-Östrogene, Verfahren zu deren Herstellung und deren Verwendung zur Weiterverarbeitung.**

(30) Priorität: **21.07.78 DE 2832604**

(43) Veröffentlichungstag der Anmeldung:
**06.02.80 Patentblatt 80/3**

(45) Bekanntmachung des Hinweises auf die Patenterteilung:
**28.10.81 Patentblatt 81/43**

(84) Benannte Vertragsstaaten:
**AT BE CH DE FR GB IT LU NL SE**

(56) Entgegenhaltungen:
**DE-A-1 793 617**
**DE-A-1 812 123**
**FR-A-1 374 699**
**US-A-4 066 674**

**CHEMISCHE BERICHTE, Band 110, 3. August 1977, Weinheim, DE, ULRICH EDER et al.: «Synthese des (+)-1,3-Dimethoxy 7β-methyl-1,3,5(10)-Ostratrien-17-ons»**

(73) Patentinhaber: **SCHERING AKTIENGESELLSCHAFT**
**Berlin und Bergkamen,**
**Müllerstrasse 170/178 Postfach 65 0311,**
**D-1000 Berlin 65 (DE)**

(72) Erfinder: **Eder, Ulrich, Dr., Zeltinger Strasse 17, D-1000 Berlin 28 (DE)**
Erfinder: **Haffer, Gregor, Dr., Alt Mariendorf 36, D-1000 Berlin 42 (DE)**
Erfinder: **Neef, Günter, Dr., Darmstädterstrasse 9, D-1000 Berlin 15 (DE)**
Erfinder: **Sauer, Gerhard, Dr., Königsbacher Zeile 41 A, D-1000 Berlin 28 (DE)**
Erfinder: **Wiechert, Rudolf, Prof. Dr., Petzower Strasse 8A, D-1000 Berlin 39 (DE)**

**EP 0 007 516 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 7α-Methyl-Östrogene, Verfahren zu deren Herstellung und deren Verwendung zur Weiterverarbeitung

Die Erfindung betrifft die in den Ansprüchen gekennzeichneten Gegenstände.

Die 7α-Methyl-1,3,5(10),9-östratetraene der allgemeinen Formel I sind neue Verbindungen, die als Zwischenprodukte zur Herstellung von pharmakologisch wirksamen Steroiden verwendet werden können.

Unter niederen Alkylgruppen $R_1$, $R_2$ und $R_3$ sollen Alkylgruppen mit 1–4 C-Atomen verstanden werden, wie beispielsweise Methyl, Äthyl, Propyl, Isopropyl, Butyl, wobei die Methyl- und Äthylgruppe bevorzugt ist.

Liegt $R_3$ in der Bedeutung eines gegebenenfalls substituierten Arylrestes vor, so ist der Phenylrest als bevorzugt anzusehen. Der Phenylrest kann auch zusätzlich substituiert sein, vorzugsweise durch eine Methylgruppe. Als funktionell geschützte Hydroxymethylengruppe X kommen Ester- und Ätherreste in Betracht. Vorzugsweise sind Alkoxymethylengruppen mit 1 bis 4 Kohlenstoffatomen als Schutzgruppen geeignet, beispielsweise seien genannt: der Methoxy-, Äthoxy-, Propyloxy-, Butyloxy- oder der Isopropyloxyrest, wobei der tert.-Butyloxyrest bevorzugt ist.

Die Herstellung der neuen Verbindungen nach dem erfindungsgemässen Verfahren und die Weiterreaktion zu den pharmakologisch wirksamen Verbindungen erfolgt in so guten Ausbeuten und so wenigen Reaktionsschritten, dass in unerwarteter Weise eine ökonomische Herstellung dieser wichtigen Verbindungen ermöglicht wird.

Für den Reaktionsschritt a) des erfindungsgemässen Verfahrens verwendet man als Deprotonierungsmittel bevorzugt Alkalihydride, Alkalialkoholate, Alkaliamide u.a., beispielsweise Natriumhydrid, Natriumamid, Kaliumhydrid, Na-tert.-butylat, Lithium-amid, Triphenylmethylkalium usw. Die Reaktion wird in einem inerten Lösungsmittel durchgeführt. Geeignete Lösungsmittel sind beispielsweise polare Äther, wie 1,2-Dimethoxyäthan, 2',2'-Dimethoxy-diäthyläther, Tetrahydrofuran oder Dioxan, sekundäre oder tertiäre Alkohole, wie Isopropanol, Butanol-(2) oder tertiär-Butanol oder dipolare aprotische Lösungsmittel, wie Dimethylformamid, N,N-Dimethylacetamid, N-Methylpyrrolidon oder Hexamethylphosphorsäuretriamid. Andererseits ist es aber auch möglich, für diese Reaktion Lösungsmittelgemische aus den obengenannten Lösungsmitteln und relativ unpolaren Lösungsmitteln, wie Benzol oder Toluol, anzuwenden.

Für diesen Reaktionsschritt ist es vorteilhaft, bei Raumtemperatur bis zur Siedetemperatur des Lösungsmittels zweckmässigerweise unter einer Schutzgasatmosphäre wie beispielsweise Argon oder Stickstoff zu arbeiten.

Mit oder vorzugsweise nach Zugabe des Deprotonierungsmittels wird die Reaktionsmischung mit einem Tosylat der allgemeinen Formel II umgesetzt. Diese Umsetzung erfolgt vorzugsweise bei einer Reaktionstemperatur von +10°C bis 80°C.

Es ist für den Fachmann überraschend, dass der Reaktionsschritt a) unter vollständiger Inversion abläuft, so dass man in Alkylierungsausbeuten von 60–80% das gewünschte Diastereoisomere isolieren kann.

Als Edelmetallkatalysatoren in der Verfahrensstufe b) kommen Pd- und Pt-Katalysatoren gegebenenfalls auf Trägern wie beispielsweise $CaCO_3$, $SrCO_3$, $BaSO_4$, Aktivkohle u.a. in Betracht, wobei insbesondere Pd-Kohle bevorzugt ist.

Die Hydrierung kann in protischen Lösungsmitteln wie z.B. Methanol, Äthanol, n-Propanol, i-Propanol, n-Butanol u.a. durchgeführt werden.

Zweckmässigerweise erfolgt die Hydrierung unter Zusatz eines tert. Amines wie z.B. Trimethylamin, Triäthylamin, Tri-n-propylamin, Dimethylamin, Isopropyl-diäthylenamin etc.

Die Hydrierung kann sowohl bei Raumtemperatur als auch bei erhöhter Temperatur durchgeführt werden; bevorzugt ist eine Reaktionstemperatur von 0°C bis 50°C.

Die Hydrierung kann sowohl unter Normaldruck als auch bei erhöhtem Druck vorgenommen werden. Vorzugsweise erfolgt die Hydrierung bei 1–80 Atm.

Da bei der Hydrierung auch eine teilweise Reduktion der 5-Keto-Gruppe in eine 5-Hydroxy-Gruppe eintreten kann, gibt man zweckmässigerweise zu dem erhaltenen Reaktionsgemisch ein Oxidationsmittel wie z.B. Jones-Reagenz, um eine Reoxidation der gegebenenfalls entstandenen 5-Hydroxygruppe zu erreichen.

Als Hydrierungsprodukt und gegebenenfalls nach Reoxidation resultiert ein Gemisch von 3aα, 4α- und 3aα, 4β-Stereoisomeren der allgemeinen Formel V. Durch Äquilibrierung des Isomerengemisches können die Verbindungen mit der erwünschten 3aα, 4β-Konfiguration erhalten werden. Für die Äquilibrierung ist es nicht notwendig, das Gemisch der Stereoisomeren zu trennen. Die Äquilibrierung kann mit an sich bekannten Mitteln vorgenommen werden, z.B. durch Behandeln mit Basen wie beispielsweise Alkalimetallalkoholaten wie zum Beispiel Na-Methylat in einem inerten Lösungsmittel wie zum Beispiel niedere Alkohole z.B. Methanol.

Die Äquilibrierung kann auch mittels Chromatographie vorgenommen werden, z.B. unter Verwendung einer Kolonne mit basischen Eigenschaften, wie einer Aluminiumoxid-Kolonne. Für den Reaktionsschritt b) ist es überraschend, dass die Hydrierung im Sinne einer trans-Hydrierung bezogen auf die C/D-Ringverknüpfung abläuft, da der Einfluss einer 7-Methylgruppe nicht vorhersehbar war.

Die Cyclisierung zu den gewünschten Endprodukten der allgemeinen Formel I kann gleichzeitig mit der Äquilibrierung oder im Anschluss daran durchgeführt werden. Die Cyclisierung erfolgt in der Regel mit besseren Ausbeuten, wenn die Äquilibrierung gesondert vorgenommen wird.

Als Cyclisierungsmittel sind vorzugsweise saure Katalysatoren wie beispielsweise Mineralsäuren, Sulfonsäure, Lewissäuren oder stark dissoziierende Carbonsäuren. Beispielsweise seien genannt: Ameisensäure, Monofluoressigsäure, Trichloressigsäure, Oxalsäure, Malonsäure, Salzsäure, Bromwasserstoffsäure, Schwefelsäure, Phosphorsäure, Perchlorsäure, Methansulfonsäure, Benzolsulfonsäure, p-Toluolsulfonsäure oder Bortrifluorid. Besonders bevorzugt sind Mineralsäure, wie beispielsweise Salzsäure, Schwefelsäure und Phosphorsäure.

Geeignete organische Lösungsmittel für die Cyclisierung sind z.B. Alkohole, Äther, Ketone, Kohlenwasserstoffe, chlorierte Kohlenwasserstoffe u.a., wobei Alkohole wie zum Beispiel Äthanol, Propanol, Butanol u.a., insbesondere jedoch Methanol als bevorzugt anzusehen sind. Die Cyclisierung kann sowohl bei tiefer Temperatur – etwa ab −10°C – als auch bei erhöhter Temperatur – bis etwa 150°C – durchgeführt werden. Vorzugsweise erfolgt die Cyclisierung bei einer Reaktionstemperatur von +0°C bis +70°C.

Inwieweit durch die Einführung einer 7α-Methylgruppe der Cyclisierungsablauf dahingehend beeinflusst wird, dass neben $\Delta^{9(11)}$-Verbindungen auch $\Delta^{8(9)}$-Verbindungen erhalten werden, liess sich auch von einem Fachmann nicht vorhersehen. Es ist daher überraschend, dass als Cyclisierungsprodukt nahezu quantitativ die gewünschten 7α-Methyl-1,3,5(10),9-Östratetraene isoliert werden können. Die Freisetzung der funktionell geschützten Hydroxymethylengruppe X erfolgt nach dem Fachmann bekannten Verfahren z.B. durch saure Hydrolyse.

Die neuen Verbindungen der allgemeinen Formel I eignen sich insbesondere zur Herstellung von 7α-Methyl-östratrienen, die als Zwischenprodukte für pharmakologisch wirksame Steroide bekannt sind (Dt. Patent 1 593 509).

Die Reduktion der $\Delta^{9(11)}$-Doppelbindung kann nach an sich bekannten Methoden erfolgen, beispielsweise durch eine Birch-Reduktion. Diese wird in flüssigem Ammoniak und einem inerten Lösungsmittel wie zum Beispiel THF, Dioxan, Dimethoxyäthan oder Äther mit Lithium, Na oder Calcium durchgeführt, wobei zweckmässigerweise um eine Reduktion des aromatischen Ringes zu vermeiden, Anilin, Monomethylanilin oder eine ähnliche Verbindung zugesetzt wird. Geeignete Reaktionstemperaturen liegen zwischen −70°C bis −35°C. Überraschenderweise läuft die Reduktion stereochemisch einheitlich ab und man isoliert nur eine Verbindung mit 9α-H-Konfiguration. Die katalytische Hydrierung liefert ganz überwiegend die unnatürliche 9βH-Konfiguration.

Die vorliegende Erfindung betrifft daher auch die Verwendung der Verbindungen der allgemeinen Formel I zur Herstellung von Verbindungen der allgemeinen Formel IX

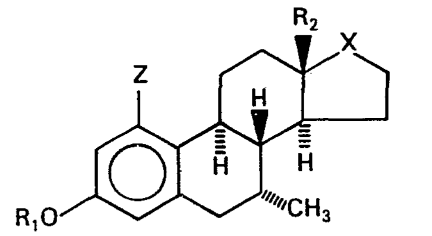

(IX)

worin $R_1$, $R_2$, Z und X die obige Bedeutung haben.

Die Überführung der Verbindungen der allgemeinen Formel IX in pharmakologisch wirksame Verbindungen insbesondere mit östrogener und ovulationshemmender Wirkung erfolgt nach an sich bekannten Verfahren.

Beispielsweise wird die funktionell geschützte 17β-Hydroxymethylengruppe X in eine freie Hydroxymethylengruppe überführt und anschliessend zur 17-Keto-Verbindung oxidiert. Anschliessend kann der 1,3-Diäther – wie bekannt – zu einem 7α-Methyl-$\Delta^{1,3,5,(10)}$-östratrien-17-on-1,3-diacetat umgewandelt werden, dessen Herstellung und Weiterverarbeitung in der Dt-Patentschrift 1 593 509 beschrieben ist.

Die Herstellung der neuen Ausgangsverbindungen der allgemeinen Formel II kann nach an sich bekannten Methoden erfolgen. Beispielsweise wird 1-(3,5-Dialkoxyphenyl)-2-propanon mit $NaBH_4$ zum 2-Propanol reduziert und anschliessend in üblicher Weise mit einer optisch aktiven Base wie Brucin die Racemat-Trennung vorgenommen.

Die Verbindung der allgemeinen Formel II'

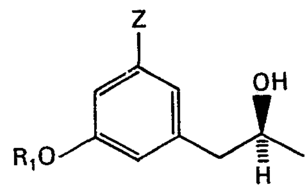

(II'),

worin $R_1$ und Z die obige Bedeutung haben, kann auch auf mikrobiologischem Wege hergestellt werden.

Der Alkohol der Formel II' kann anschliessend in üblicher Weise in den Sulfonsäureester der allgemeinen Formel II überführt werden.

Herstellung der neuen Ausgangsverbindungen

A. p-Toluolsulfonsäure-[(S)-(+)-1-(3,5-dimethoxy-phenylmethyl)-äthyl]-ester (S)-(+)-3

1. 43,7 g 1-(3,5-Dimethoxyphenyl)-2-propanon (A.J. Birch und F.W. Donovan, Australien J. Chem. 6, 373 (1953)) werden in 430 ml Äthanol gelöst und bei Eiskühlung mit 2,65 g Natriumborhydrid anteilweise versetzt. Nach 3 Stunden bei 0°C wird mit 1 n Schwefelsäure bis pH 5 angesäuert, das Äthanol im Vakuum weitgehend abdestilliert, 200 ml Wasser zugefügt und mit Methylenchlorid extrahiert. Nach Neutralwaschen und Trocknen mit Natriumsulfat wird das Lösungsmittel im Vakuum abdestilliert.

Man erhält 42,32 g (R,S)-1-(3,5-Dimethoxyphenyl)-2-propanol als farbloses Öl vom Siedepunkt 120–123° bei 0,03 Torr.

2. a) Racemat-Trennung von (±)-2 über das Hemiphthalat und Salzbildung mit Brucin

Zur Lösung von 51 g (R,S)-1-(3,5-Dimethoxyphenyl)-2-propanol in 40 ml Pyridin fügt man 38,6 g Phthalsäureanhydrid und erhitzt für 2 Stunden auf 100°C. Die abgekühlte Mischung wird auf 200 g Eis und 200 ml 5 n Salzsäure gegossen und mit Chloroform mehrfach extrahiert. Die vereinigten Chloroform-Extrakte werden dreimal mit je 300 ml gesättigter Sodalösung extrahiert. Die vereinigten Sodaextrakte werden bei intensiver Eiskühlung mit 5 n Salzsäure bis pH 1 angesäuert, mit Chloroform extrahiert und die Chloroformextrakte mit halbgesättigter Kochsalzlösung neutral gewaschen und anschliessend getrocknet. Nach Abdestillation des Lösungsmittels im Vakuum verbleiben 88,5 g Phthalsäurehalbester als gelbes Öl. Dieses Rohprodukt wird in 600 ml Aceton gelöst, mit 120 g Brucin versetzt und für 20 Minuten gekocht. Bei Abkühlen kristallisieren 95,3 g linksdrehendes Brucin-Salz aus, das nach erneuter Kristallisation aus Aceton 87,6 g Brucin-Salz vom Schmelzpunkt 165–167°C und $[\alpha]^{23}_D$ = −23,7° (c=1, $CHCl_3$) ergibt.

Die Suspension von 87,1 g dieses Brucin-Salzes in 330 ml Äther und 330 ml Wasser wird mit 60 ml Salzsäure versetzt und für 30 Minuten heftig bei Raumtemperatur gerührt, dann wird die Wasserphase abgetrennt und dreimal mit je 100 ml Äther extrahiert. Die vereinigten Ätherphasen werden mit halbgesättigter Kochsalzlösung neutralgewaschen, mit $Na_2SO_4$ getrocknet und das Lösungsmittel im Vakuum abdestilliert.

Der hellgelbe ölige Rückstand [40,2 g, $[\alpha]^{23}_D$ + 26,1° (c= 1, $CHCl_3$)] wird in 200 ml 2 n Natronlauge gelöst, 2 Stunden gekocht, abgekühlt und mit Äther extrahiert. Nach Neutralwaschen und Trocknen und Abdestillieren des Äthers erhält man 24,3 g hellgelbes Öl, das im Hochvakuum destilliert wird.

Ausbeute 22,3 g (S)-(+)-1-(3,5-Dimethoxyphenyl)-2-propanol vom Siedepunkt 123–126°/0,08 Torr und einem $[\alpha]^{23}_D$ + 29,1° (c= 1, $CHCl_3$).

3. p-Toluolsulfonsäure-[(S)-(+)-1-(3,5-dimethoxyphenylmethyl)-äthyl]-ester (S)-(+)-3

10,2 g (S)-(+)-1-(3,5-Dimethoxyphenyl)-2-propanol werden in 80 ml Pyridin gelöst, auf 0°C abgekühlt und anteilweise mit 14,5 g p-Toluolsulfonsäurechlorid versetzt. Nach 16 Stunden bei +5° wird auf 300 g Eis und 200 ml 2n Salzsäure gegossen, mit Methylenchlorid extrahiert und anschliessend neutralgewaschen und getrocknet. Das Rohprodukt kann so weiter verarbeitet werden oder aber auch chromatographiert werden an Kieselgel mit Benzin-Aceton 0–40%. Man erhält (nach Chromatographie) 15,2 g p-Toluolsulfonsäure-[(S)-(+)-1-(3,5-dimethoxyphenylmethyl)-äthyl]-ester als farbloses Öl. $[\alpha]^{23}_D$ + 25,3° (c=1, $CHCl_3$).

B. p-Toluolsulfonsäure-[S-(+)-1-(3-methoxyphenylmethyläthyl]-ester

1. (R,S)-1-(3-Methoxyphenyl)-2-propanol

67,3 g 1(3-Methoxyphenyl)-2-propanon (Z. Horii et al., J. Chem. Soc. (London) 1963, 3940) werden nach A in Äthanol mit Natriumborhydrid reduziert. Es werden 59,67 g (R,S)-1-(3-Methoxyphenyl)-2-propanol vom Siedepunkt 110–112°/ 0,06 Torr erhalten.

2. Die Racemat-Trennung von (R,S)-1-(3-Methoxyphenyl)-2-propanol erfolgt analog A. 2.

59,3 g racemischer Alkohol ergeben 89,4 g linksdrehendes Brucin-Salz vom Schmelzpunkt 143–146°C und $[\alpha]^{23}_D$ = −19,2° (c=1, $CHCl_3$), das nach Weiterverarbeitung 19,6 g optisch aktives (S)-(+)-1-(3-Methoxyphenyl)-2-propanol (farbloses Öl) vom Siedepunkt 117–119°C bei 0,05 Torr ergibt.

$[\alpha]^{23}_D$ = +23,4° (c=1, $CHCl_3$).

3. p-Toluolsulfonsäure-[S-(+)-1-(3-methoxyphenylmethyl)-äthyl]-ester

Die Umsetzung erfolgt analog A, 3.)

6,7 g (S)-(+)-1-(3-Methoxyphenyl)-2-propanol ergeben 9,15 g p-Toluolsulfonsäureester als farbloses Öl nach chromatographischer Reinigung.

Beispiel 1

a) (1S,7aS)-(+)-1-tert.-Butoxy-4-[(1R)-2-(3,5-dimethoxyphenyl)-1-methyläthyl]-7a-methyl-2,3,5,6,7,7a-hexahydro-1H-inden-5-on

15 g (1S,7aS)-(+)-1-tert. Butoxy-7a-methyl-5,6,7,7a-tetrahydroindan-5-on werden mit 1,62 g Natriumhydrid in 150 ml absolutem Dimethoxyäthan unter Argonschutzgas für 20 Stunden auf 60°C erwärmt. Dann werden 22,0 g Tosylat aus der vorhergehenden Stufe gelöst in 100 ml Dimethoxyäthan, bei 60°C inneralb von 10 Minuten zugetropft. Nach 16 Stunden bei 80°C wird abgekühlt, mit 150 ml gesättigter Natriumdihydrogenphosphatlösung versetzt, das Dimethoxyäthan im Vakuum abdestilliert und der Rückstand mit Methylenchlorid extrahiert. Das nach Trocknen und Abdestillieren des Lösungsmittels erhaltene Rohprodukt wird an 1 kg Kieselgel mit Hexan/ Aceton (0–8%) zur Reinigung chromatographiert. Man erhält 17,7 g als farbloses Öl.

CD-Spektrum: 354 mm (△ε + 2,40), 341 (+ 2,65) 331 (+ 2,09), 317 (+1,26), 245 (−12,7)
UV-Spektrum: 296 mm (ε = 1350), 258 (11 800), 219 (36 500).

b) (1S,3aS,4S,7aS)-1-tert. Butoxy-4[(1R)-2-(3,5-dimethoxyphenyl)-1-methyläthyl]-7a-methyl-perhydroinden-5-on

4,27 g (1S,7aS)-(+)-1-tert. Butoxy-4-[(1R)-2-(3,5-dimethoxyphenyl)-1-methyläthyl]-7a-methyl-2,3,5,6,7,7a-hexahydro-1H-inden-5-on werden in 90 ml Äthanol und 0,43 ml Triäthylamin gelöst und nach Zugabe von 2,56 g Pd-Kohle (10%) bei Raumtemperatur und 60 Atmosphären Wasserstoffdruck hydriert. Hydrierzeit: 24 Stunden. Es wird vom Katalysator abfiltriert und das Lösungsmittel im Vakuum abdestilliert. Der Rückstand wird in 130 ml Aceton gelöst, auf −5°C abgekühlt und tropfenweise mit 2,7 ml 8n-Chromsäure (Jones-Lösung) versetzt. Nach 10 Minuten wird in 250 ml Wasser eingerührt und mit Methylenchlorid extrahiert. Nach Neutralwaschen und Trocknen wird das Lösungsmittel im Vakuum abdestilliert. Das erhaltene Rohprodukt (4,09 g) wird zur Äquilibrierung der Seitenkette mit 800 mg Natriummethylat in 80 ml Methanol für 2 Stunden gekocht. Man erhält nach chromatographischer Reinigung an 400 g Kieselgel mit Benzin/Aceton (0–10%) 3,75 g der Titelverbindung als dünnschichteinheitliches Öl.

CD-Spektrum: 317 mm ($\Delta\varepsilon$ + 0,725), 300 (+ 1,36) 284 ($\Delta\varepsilon$ + 0,938), 241 (+ 0,256)
$[\alpha]^{23}_D$ + 27,1° (c= 0,5 $CHCl_3$).

c) 17β-tert. Butoxy-1,3-dimethoxy-7α-methyl-1,3,5(10),9-östratetraen

30,3 g (1S,3aS,4S,7aS)-1-tert. Butoxy-5[(1R)-2-(3,5-dimethoxyphenyl)-1-methyläthyl]-7a-methyl-perhydroinden-5-on in 400 ml Methanol wird auf 10°C abgekühlt und innerhalb von 20 Minuten mit 80 ml 10 n HCl versetzt. Nach 30 Minuten beginnt das Cyclisierungsprodukt auszufallen; nach einer Zeit von insgesamt 6 Stunden bei 10° wird das ausgefallene Produkt abfiltriert, mit wenig eiskaltem Methanol gewaschen und im Vakuum bei 50°C für 12 Stunden getrocknet. Man erhält 27,36 g 17β-tert-Butoxy-1,3-dimethoxy-7α-methyl-1,3,5(10),9(11)-östratetraen vom Schmelzpunkt 143–145°C (aus Methanol), $[\alpha]^{23}_D$ + 82,5° (c = 0,5 $CHCl_3$).
UV-Spektrum: 304 mm ($\varepsilon$ = 3380), 292 (4190) 265 (19800), 221 (30400).

Beispiel 2

17β-tert.-Butoxy-1,3-dimethoxy-7α-methyl-1,3,5(10)-östratrien

9,8 g 17β-tert-Butoxy-1,3-dimethoxy-7α-methyl-1,3,5(10),9)11)-östratetraen, gelöst in 250 ml absolutem Tetrahydrofuran, werden bei −60°C innerhalb von 10 Minuten zu 500 ml flüssigem Ammoniak und 20 ml frisch destilliertem Anilin gegeben. Dann wird innerhalb von 20 Minuten 1 g Lithium anteilweise zugefügt, für 5 Minuten bei −60°C nachgerührt und anschliessend 9 g Ammoniumchlorid in 9 Portionen zugegeben. Nach Abdampfen des Ammoniaks wird der Rückstand mit 500 ml Essigester versetzt und der Essigesterextrakt mit Wasser, eiskalter In Schwefelsäure und Wasser ausgewaschen. Nach Trocknen mit Natriumsulfat und Abdestillieren des Lösungsmittels im Vakuum verbleiben 9,95 g Rohprodukt, das aus Methanol umkristallisiert wird. Man erhält 8,67 g 17β-tert-Butoxy-1,3-dimethoxy-7α-methyl-1,3,5(10)-östratrien vom Schmelzpunkt 135–135°C; $[\alpha]^{23}_D$ + 126° (c=0,5 $CHCl_3$).
CD-Spektrum: 282 mm ($\varepsilon$ + 1,56), 277 (+ 1,54) 239 (+ 6,90), 223 (−0,445).
UV-Spektrum: 286 mm ($\varepsilon$ = 1950), 282 (1880), 278 (1890), 226 (10600), 205 (52700).

Beispiel 3

(1S,7aS)-(+)-1-tert.-Butoxy-4-[(1R)-2-(3-methoxyphenyl)-1-methyläthyl]-7a-methyl-2,3,5,6,7,7a-hexahydro-1H-inden-5-on

8,3 g (1S,7aS)-(+)-1-tert.-Butoxy-7a-methyl-5,6,7,7a-tetrahydroindan-5-on werden mit 12,8 g p-Toluolsulfonsäure-[S-(+)-1-(3-methoxyphenylmethyl)-äthyl]-ester analog Beispiel 1 a) umgesetzt. Nach chromatographischer Reinigung an Kieselgel mit Hexan/Aceton (0–6%) werden 10,1 g Alkylierungsprodukt als farbloses Öl erhalten. UV-Spektrum: 293 nm ($\varepsilon$ = 1270), 255 (11950), 221 (33800). IR-Spektrum: 1693 $mm^{-1}$ (α,β-ungesättigtes Keton).

b) (1S,3aS,4S,7aS)-1-tert.-Butoxy-4-[(1R)-2-(3-methoxyphenyl)-1-methyläthyl]-7a-methyl-perhydroinden-5-on

Nach Hydrierung, Oxidation und Äquilibrierung analog Beispiel 1 b) werden aus 9,95 g Produkt aus Stufe 3 a) 7,45 g der Titelverbindung vom Schmelzpunkt 45–47°C erhalten $[\alpha]^{23}_D$ = +30,3° (c= 0,5 $CHCl_3$).

c) 17β-tert.-Butoxy-methoxy-7α-methyl-1,3,5(10),9(11)-östratetraen

84,2 g Produkt aus Stufe 3 b) werden analog Beispiel 1 c) in 842 ml Methanol mit 160 ml 10n Salzsäure bei +10°C/+15°C cyclisiert. Die Reaktionszeit beträgt 20 Stunden. Das ausgefallene Produkt wird abfiltriert und mit wenig eiskaltem Methanol gewaschen. Nach Kristallisation aus Methanol erhält man 68,79 g der Titelverbindung vom Schmelzpunkt 138–141°C.

Beispiel 4

17β-Hydroxy-3-methoxy-7α-methyl-1,3,5(10)-östratrien

Birch-Reduktion nach Beispiel 2, aber bei −40°C. 11,2 g 17β-Butoxy-3-methoxy-7α-methyl-1,3,5(10),9(11)-östratetraen werden analog Beispiel 2 bei −40°C umgesetzt und man erhält 10,63 g rohes 17β-tert.-Butoxy-3-methoxy-7α-methyl-1,3,5(10)-östratrien, das in 250 ml Dioxan und 50 ml 4n Salzsäure für 3,5 Stunden unter Rückfluss gekocht wird. Das Dioxan wird weitgehend im Vakuum abdestilliert, der Rückstand mit 300 ml Wasser verdünnt und mit Methylenchlorid extrahiert. Nach Neutralwaschen mit Wasser und Trocknen mit Natriumsulfat wird das Lösungsmittel im Vakuum abdestilliert. Der kristalline Rückstand wird aus Methanol/Tetrahydrofuran umkristallisiert. Es resultieren 6,93 g 17β-Hydroxy-3-methoxy-7α-methyl-1,3,5(10)-östratrien.

Schmelzpunkt: 128–130°C.

**Patentansprüche**

für die Vertragsstaaten: BE, CH, DE, FR, GB, IT, LU, NL, SE

1.) 7α-Methyl-Östrogene der allgemeinen Formel I

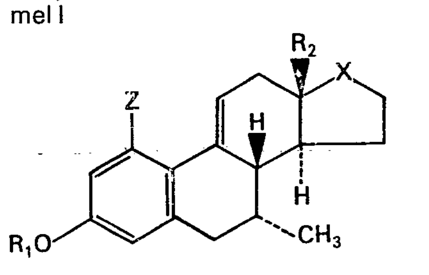

(I)

worin
$R_1$ und $R_2$ niedere Alkylgruppen,
X eine feste oder funktionell geschützte Hydroxymethylengruppe und
Z ein Wasserstoffatom oder eine $OR_1$-Gruppe bedeutet.

2.) Verfahren zur Herstellung von Verbindungen der allgemeinen Formel I, dadurch gekennzeichnet, dass man in an sich bekannter Weise
a) eine Verbindung der allgemeinen Formel II

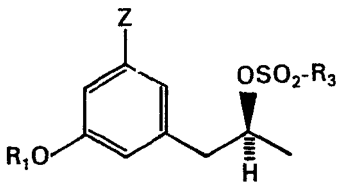
(II),

worin $R_1$ und Z die obige Bedeutung haben und $R_3$ eine niedere Alkyl- oder eine gegebenenfalls substituierte Arylgruppe bedeuten, in Gegenwart eines Deprotonierungsmittels mit einer Verbindung der allgemeinen Formel III

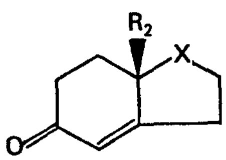
(III)

worin X und $R_2$ die obengenannte Bedeutung haben, umsetzt, wobei eine Verbindung der allgemeinen Formel IV

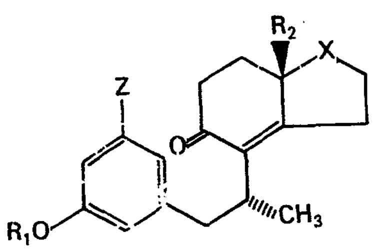
(IV)

worin $R_1$, $R_2$, Z und X die obige Bedeutung haben, erhalten wird;

b) die Verbindung der allgemeinen Formel IV mit einem Edelmetallkatalysator zu einer Verbindung der allgemeinen Formel V

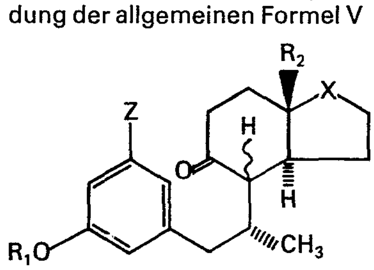
(V),

worin $R_1$, $R_2$, Z und X die obige Bedeutung haben, hydriert und

c) das Hydrierungsprodukt der allgemeinen Formel V, gegebenenfalls nach Zusatz eines Oxidationsmittels der Äquilibrierung und Cyclisierung unterwirft und gegebenenfalls Schutzgruppen abspaltet.

3.) Verwendung der Verbindungen der allgemeinen Formel I zur Herstellung von Verbindungen der allgemeinen Formel IX

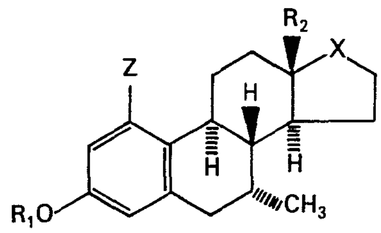
(IX),

worin $R_1$, $R_2$, Z und X die obige Bedeutung haben.

**Patentansprüche**
für den Vertragsstaat: AT

1.) Verfahren zur Herstellung von Verbindungen der allgemeinen Formel I

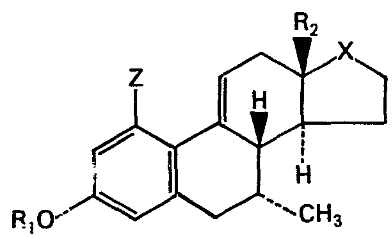
(I)

worin
$R_1$ und $R_2$ niedere Alkylgruppen,
X eine freie oder funktionell geschützte Hydroxymethylengruppe und
Z ein Wasserstoffatom oder eine $OR_1$-Gruppe bedeutet, dadurch gekennzeichnet, dass man in an sich bekannter Weise
a) eine Verbindung der allgemeinen Formel II

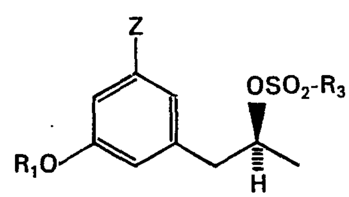
(II),

worin $R_1$ und Z die obige Bedeutung haben und $R_3$ eine niedere Alkyl- oder eine gegebenenfalls substituierte Arylgruppe bedeuten, in Gegenwart eines Deprotonierungsmittels mit einer Verbindung der allgemeinen Formel III

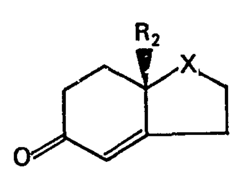
(III)

worin X und $R_2$ die obengenannte Bedeutung haben, umsetzt, wobei eine Verbindung der allgemeinen Formel IV

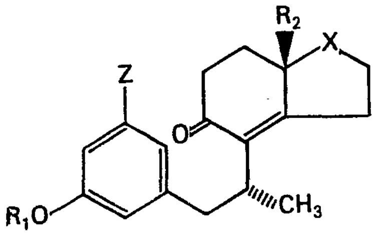

(IV)

worin $R_1$, $R_2$, Z und X die obige Bedeutung haben, erhalten wird;

b) die Verbindung der allgemeinen Formel IV mit einem Edelmetallkatalysator zu einer Verbindung der allgemeinen Formel V

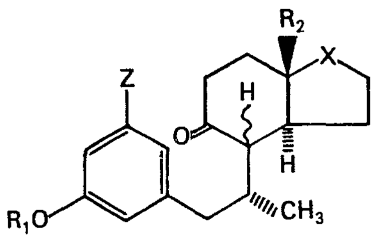

(V),

worin $R_1$, $R_2$, Z und X die obige Bedeutung haben, hydriert und

c) das Hydrierungsprodukt der allgemeinen Formel V, gegebenenfalls nach Zusatz eines Oxidationsmittels der Äquilibrierung und Cyclisierung unterwirft und gegebenenfalls Schutzgruppen abspaltet.

2.) Verwendung der Verbindungen der allgemeinen Formel I zur Herstellung von Verbindungen der allgemeinen Formel IX

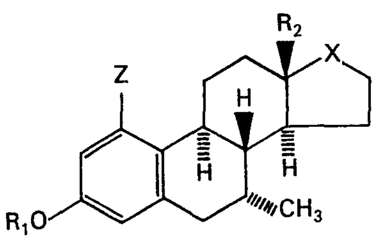

(IX),

worin $R_1$, $R_2$, Z und X die obige Bedeutung haben.

**Claims**

for the Contracting states: BE, CH, DE, FR, GB, IT, LU, NL, SE

1.) 7α-methyl oestrogens of the general formula I

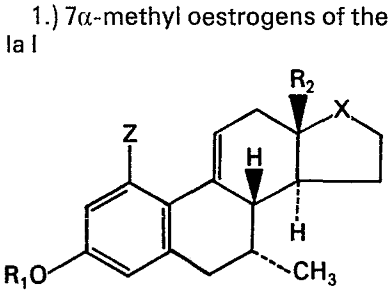

(I)

in which

$R_1$ and $R_2$ denote lower alkyl groups,

X denotes a free or functionally protected hydroxymethylene group and

Z denotes a hydrogen atom or an $OR_1$ group.

2.) Process for the manufacture of compounds of the general formula I, characterised in that, in a manner known per se,

a) a compound of the general formula II

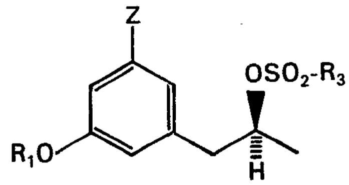

(II),

in which $R_1$ and Z have the meanings given above and $R_3$ denotes a lower alkyl group or an optionally substituted aryl group, is reacted, in the presence of a deprotonating agent, with a compound of the general formula III

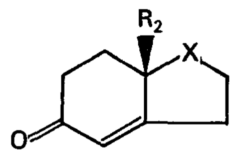

(III)

in which X and $R_2$ have the meanings given above, a compound of the general formula IV being obtained

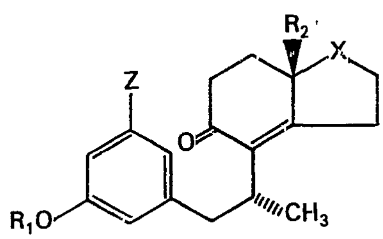

(IV)

in which $R_1$, $R_2$, Z and X have the meanings given above;

b) the compound of the general formula IV is hydrogenated with a noble metal catalyst to form a compound of the general formula V

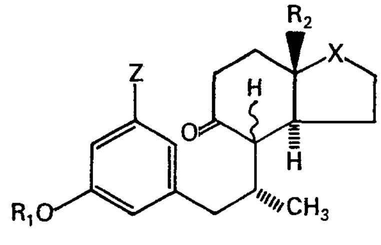

(V),

in which $R_1$, $R_2$, Z and X have the meanings given above and

c) the hydrogenation product of the general formula V, optionally after the addition of an oxidising agent; is subjected to equilibration and cyclisation and optionally protecting groups are split off.

3.) Use of the compounds of the general formula I for the manufacture of compounds of the general formula IX

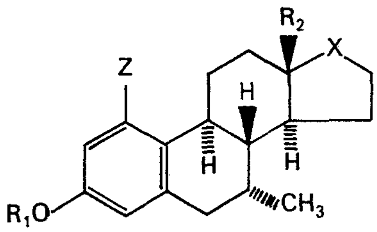

(IX),

in which $R_1$, $R_2$, Z and X have the meanings given above.

**Claims**
for the Contracting state: AT

1.) Process for the manufacture of compounds of the general formula I,

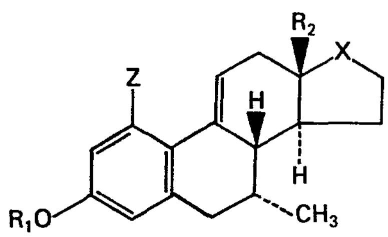

(I)

in which
$R_1$ and $R_2$ denote lower alkyl groups,
X denotes a free or functionally protected hydroxymethylene group and
Z denotes a hydrogen atom or an $OR_1$ group, characterised in that, in a manner known per se,

a) a compound of the general formula II

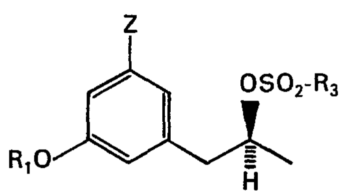

(II),

in which $R_1$ and Z have the meanings given above and $R_3$ denotes a lower alkyl group or an optionally substituted aryl group, is reacted, in the presence of a deprotonating agent, with a compound of the general formula III

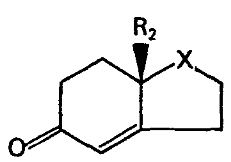

(III)

in which X and $R_2$ have the meanings given above, a compound of the general formula IV being obtained

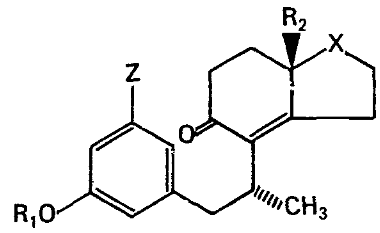

(IV)

in which $R_1$, $R_2$, Z and X have the meanings given above;

b) the compound of the general formula IV is hydrogenated with a noble metal catalyst to form a compound of the general formula V

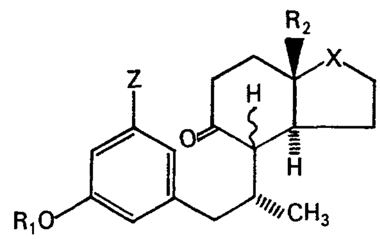

(V),

in which $R_1$, $R_2$, Z and X have the meanings given above and

c) the hydrogenation product of the general formula V, optionally after the addition of an oxidising agent, is subjected to equilibration and cyclisation and optionally protecting groups are split off.

2.) Use of the compounds of the general formula I for the manufacture of compounds of the general formula IX

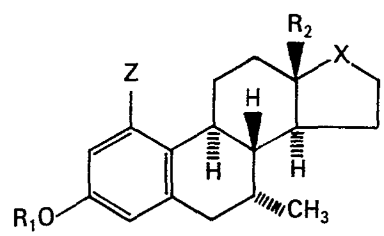

(IX),

in which $R_1$, $R_2$, Z and X have the meanings given above.

**Revendications**
pour les Etats contractants: BE, CH, OE, FR, GB, IT, LU, NL, SE

1. Méthyl-7α œstrogènes répondant à la formule générale I

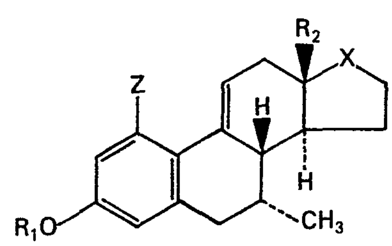

(I)

dans laquelle
$R_1$ et $R_2$ représentent des radicaux alkyles inférieurs,

X représente un radical hydroxyméthylène libre ou protégé et

Z représente un atome d'hydrogène ou un radical $-OR_1$.

2. Procédé pour préparer des composés de formule générale I, caractérisé en ce que, en opérant de manière connue:

a) on fait réagir un composé répondant à la formule générale II

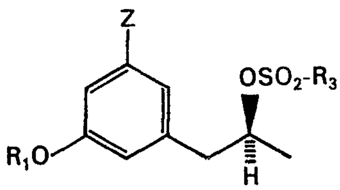

(II),

dans laquelle $R_1$ et Z ont les significations données ci-dessus et $R_3$ représente un radical alkyle inférieur ou un radical aryle éventuellement substitué, en présence d'un agent déprotonant, avec un composé répondant à la formule générale III

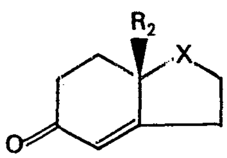

(III)

dans laquelle X et $R_2$ ont les significations précédemment données, cette réaction conduisant à un composé répondant à la formule générale IV

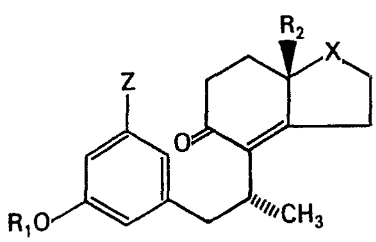

(IV)

dans laquelle $R_1$, $R_2$, Z et X ont les significations indiquées ci-dessus,

b) on hydrogène le composé de formule générale IV au moyen d'un catalyseur à base d'un métal noble de manière à obtenir un composé répondant à la formule générale V

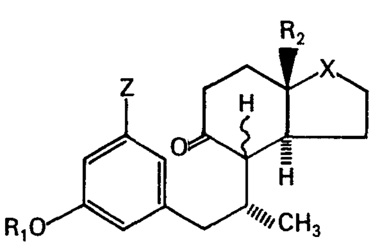

(V),

dans laquelle $R_1$, $R_2$, Z et X ont les significations précédemment données, et

c) on soumet le produit d'hydrogénation de formule générale V, éventuellement après avoir ajouté un agent d'oxydation, à un équilibrage et à une cyclisation, et éventuellement on élimine les groupes protecteurs.

3. Utilisation des composés de formule générale I pour la préparation de composés répondant à la formule générale IX

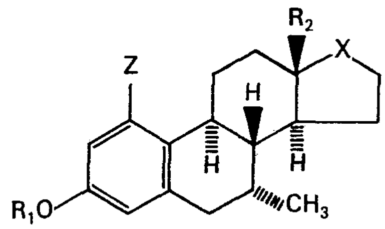

(IX),

dans laquelle $R_1$, $R_2$, Z et X ont les significations indiquées plus haut.

**Revendications**

pour l'Etat contractant: AT

1. Procédé pour préparer des composés de formule générale I,

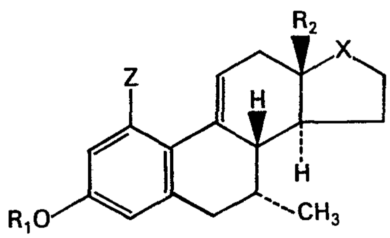

(I)

dans laquelle

$R_1$ et $R_2$ représentent des radicaux alkyles inférieurs,

X représente un radical hydroxyméthylène libre ou protégé et

Z représente un atome d'hydrogène ou un radical $-OR_1$,

caractérisé en ce que, en opérant de manière connue:

a) on fait réagir un composé répondant à la formule générale II

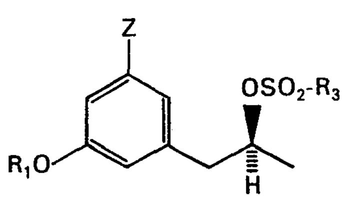

(II),

dans laquelle $R_1$ et Z ont les significations données ci-dessus et $R_3$ représente un radical alkyle inférieur ou un radical aryle éventuellement substitué, en présence d'un agent déprotonant, avec un composé répondant à la formule générale III

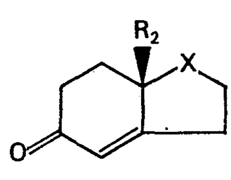

(III)

dans laquelle X et $R_2$ ont les significations précédemment données, cette réaction conduisant à un composé répondant à la formule générale IV

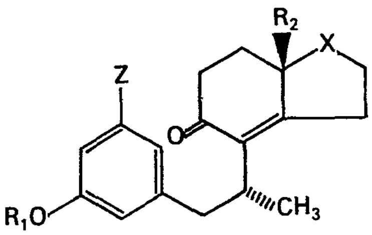

(IV)

dans laquelle $R_1$, $R_2$, Z et X ont les significations indiquées ci-dessus,

b) on hydrogène le composé de formule générale IV au moyen d'un catalyseur à base d'un métal noble de manière à obtenir un composé répondant à la formule générale V

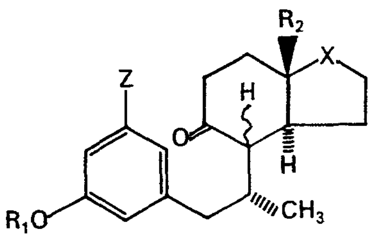

(V),

dans laquelle $R_1$, $R_2$, Z et X ont les significations précédemment données, et

c) on soumet le produit d'hydrogénation de formule générale V, éventuellement après avoir ajouté un agent d'oxydation, à un équilibrage et à une cyclisation, et éventuellement on élimine les groupes protecteurs.

2. Utilisation des composés de formule générale I pour la préparation de composés répondant à la formule générale IX

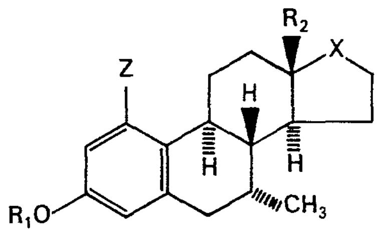

(IX),

dans laquelle $R_1$, $R_2$, Z et X ont les significations indiquées plus haut.